# EUROPEAN PATENT APPLICATION

(11) **EP 2 019 317 A1**
(43) Date of publication of application: **28.01.2009**
(21) Application number: 07113017.3
(22) Date of filing: 24.07.2007
(51) Int. Cl.: G01N 33/487, G01N 15/10, G01N 15/12, G01N 27/414

(54) **A Microfluidic System for Analyzing Particles**

(71) Applicant: Evotec AG, 22525 Hamburg (DE); Evotec Technologies GmbH, 22525 Hamburg (DE)
(72) Inventor: Möller, Clemens, 25469, Halstenbek (DE); Müller, Torsten, 12439, Berlin (DE); Schnelle, Thomas, 10318, Berlin (DE)
(74) Representative: von Kirschbaum, Alexander

(57) **Abstract**

A microfluidic system for analyzing particles, particularly biological cells, comprises a receptacle (30). In the receptacle, a fluid having particles (32) suspended therein is provided. A side wall of the receptacle is formed by a substrate (34) having at least one through opening (36). At an outer side (46) of the substrate (34), a fluid channel (48) is provided. Further, a pressure generation means (38) for generating a flow through the through opening (36) into the fluid channel (48) is provided, For performing measurements, an electrode (56,58) is provided on each side of the substrate (34).

## Description

The invention relates to a microfluidic system for analyzing particles, particularly biological cells, membranes (particularly lipid membranes), membrane fragments, vesicles (particularly lipid vesicles), lipids, cell fragments, beads (particularly labeled beads).

For the analysis of electrophysiological parameters of cells, or particularly of ion channels expressed in cells, the patch clamp technique is known. By means of a pipette (or, most recently, a planar substrate), a mechanical contact to the cell membrane is established and then, via mild suction, a high-resistance electrical contact (e.g., a giga-seal) between the cell and the pipette is established. An electrode is arranged within the pipette so that voltages or currents can be applied and/or measured against another electrode arranged outside the pipette in order to determine cell or ion channel characteristics. The patch-clamp technique is considered the gold-standard to investigate ion channel function.

For simplification, in the following, the word "cell" is used, although the same is applicable to investigate vesicles (particularly lipid vesicles), membranes (particularly lipid membranes), membrane fragments, lipids, cell fragments, beads (particularly labeled beads), etc. The patch-clamp technique allows individual or a multitude of cells to be investigated at or near physiological conditions, and allows the voltage across membranes (and thereby across transmembrane ion channels) to be controlled, which is particularly advantageous for the investigation of voltage-gated ion channels.

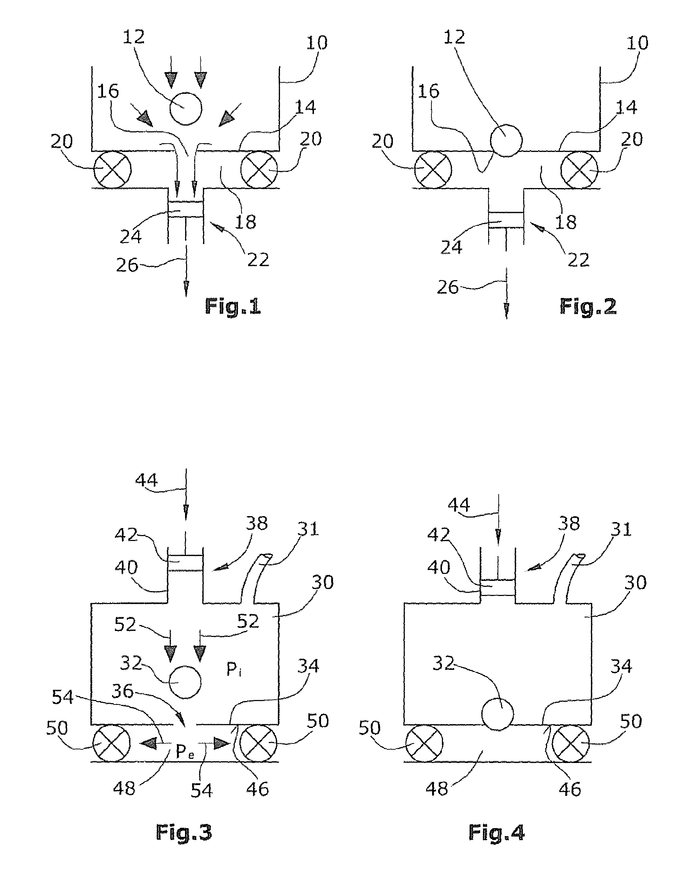

For patch-clamp investigations of cells, a high-resistance electrical seal between a cell and a substrate is established (e. g. a mega-seal or a giga-seal), thereby electrically isolating one side of the substrate from the other. As substrate, the classical patch-clamp technique uses glass pipettes. Recently, patch-clamping on planar substrates (e.g. bottom 14 of Fig. 1) has become possible. Electrical current flowing from one side of the substrate to the other thereby has to pass the cell. The cell is now in the so-called "cell-attached" configuration (cf. fig. 2). Three other configurations are widely employed for investigating ion channels or cells: (1) the "classical whole-cell", (2) the "perforated patch", and (3) the "single-channel" configuration. All these three configurations are obtained from the cell-attached configuration. Starting from the cell-attached configuration, to reach the whole-cell configuration, the membrane patch covering the opening of the isolating substrate is ruptured and thereby opened, e.g. by using voltage or pressure pulses. The cell interior is now opened to one side of the substrate, and can be manipulated. Starting from the cell-attached configuration, to reach the perforated patch configuration, the membrane patch covering the opening of the isolating substrate is opened electrically, by applying a pore-forming agent, e.g., nystatin or amphotericin. Starting from the cell-attached configuration, to reach the single channel configuration, the membrane patch covering the opening of the isolating substrate is kept intact, while the larger membrane around this is ruptured, typically by mechanical force. This allows individual channels in the membrane patch to be investigated.

Beside ion channels, secondary messenger substances also play an important role in signal transmission. Secondary messenger substances diffusing through the cytoplasm transmit signals from the cell surface into the cell interior. Many ion channels (IC), for example, are influenced and modulated by secondary messenger substances and vice versa. Therefore, it is very advantageous for numerous questions if the measuring of ionic currents, the regulator signal (e.g., Ca²⁺), and the cell response (e.g., secretion) or rather of at least two of the three variants could be performed at the same time. The whole cell patch clamp configuration permits this (since the cell contents can be manipulated) in combination with, e.g., fluorescence microscopy (e.g., the determination of Ca).

In addition, capacity measurements for characterizing endo/exocytose procedures (e.g., the activation of mast cells), and assessment of other biophysical and electrophysiological parameters, can be performed.

Further, microfluidic systems are known for analyzing particles such as cells. A schematic diagram of a known microfluidic system that is employed for automating the patch-clamp technique is illustrated in Fig. 1. The microfluidic system comprises a receptacle 10 in which a liquid having a plurality of cells suspended therein is provided. For illustrative purposes, a cell 12 is illustrated in Figs. 1 and 2. A bottom 14 of the receptacle 10 has a through opening 16. Below the through opening 16, a fluid channel 18 is provided that is closed by two valves 20. Further, the fluid channel 18 is connected to a suction means 22 for generating a negative pressure. In the illustrated example, the suction means 22 comprises a piston 24 which is drawn in the direction of an arrow 26 for generating a negative pressure in the fluid channel 18 as well as in the receptacle. This, as indicated by the arrows, produces a flow which effects a movement of the cell 12 toward the through opening 16. Thus, the cell 12 reaches the position illustrated in Fig. 2 in which it is arranged in the through opening 16. To hold the cell 12 in the through opening 16, a force acting in the direction of the arrow 26 must always be exerted onto the piston 24 to guarantee that the pressure pᵢ is at least equal, or ideally greater than the external pressure pₑ. In this position, the cell 12 may now be analyzed. To this end, non-illustrated electrodes are arranged at both sides of the bottom 14 which do not touch the cell 12. A plurality of analyses of the cell 12 require the opening of the cell membrane. The membrane of the cell can be opened by pressure and/or voltage pulses as described for the whole-cell patch-clamp technique above. Once the membrane is opened, the content of the cell can be manipulated via the fluid channel 18. This is particularly advantageous if the effect of substances onto ion channels regulated from the intracellular side are to be investigated. An alternative method for electrically opening the cells is by supplying a chemical such as nystatin via channel 18, yielding the perforated patch configuration described above. The supply of chemicals or substances to channel 18 and thereby to the intracellular volume is effected by opening the valves 20. To this end, a complex control is required in known microfluidic systems since it has to be guaranteed during the opening of the cell that the pressure pᵢ be at least equal, or ideally greater, than the pressure pₑ since, otherwise, the cell may be pressed out of the through opening in which case the cell could not be analyzed any more. Further, with such microfluidic systems, there is the danger of the formation of bubbles in the fluid suspended with cells because of the applied negative pressure. This may lead to a clogging of the microchannels.

It is the object of the invention to provide a microfluidic system and a process for analyzing particles, particularly biological cells, which has a simplified structure.

This object is solved, according to the invention, with a microfluidic system having the features of claim 1 and a process for analyzing particles having the steps of claim 18.

The microfluidic system for the analysis of particles, such as biological cells, vesicles etc. according to the invention comprises a receptacle. In the receptacle, a fluid, particularly a liquid, having particles suspended therein, is provided for the analysis. The fluid may be supplied via a supply means, a supply channel, a hose or the like. At least a portion of a side wall of the receptacle is formed by a substrate with at least one through opening. Substrates of glass and/or silicon which have pores are particularly suitable as a substrate. On an outer side of the substrate, i.e., on the side of the substrate facing away from the receptacle, a fluid channel is arranged. Thus, the receptacle is fluidly connected to the fluid channel via the through opening.

According to the invention, the receptacle is connected to a pressure generation means. According to the invention, the pressure generation means is able to generate an overpressure or flow in the receptacle. The flow passes through the through opening into the fluid channel. Due to the flow, the particles are transported toward the through opening until the through opening is closed by a particle arranged in or at the through opening. Now, this particle can be analyzed. During the analysis it is useful but not necessary to maintain the overpressure. To this end, each side of the substrate is provided with at least one electrode. By means of the electrodes, electric measurements referring to the particles arranged in or at the through opening can be performed. The known electronic measurements with respect to particles, particularly biological cells, are suitable therefore. These are, for example, the patch-clamp technique as described before using either voltage-clamp, current-clamp, capacity measurements, impedance measurements, action potential assessment, or a combination thereof.

This means that the substantial difference of the microfluidic system according to the invention to known microfluidic systems consists in that the positioning of the particle at or in the through opening is performed by means of overpressure and not by means of negative pressure. Thus, the particle is pressed into or at the through opening by a pressure generated in the receptacle. The particle is held at or in the through opening because of this overpressure prevailing in the receptacle and not by a negative pressure or by sucking the particle by means of a negative pressure applied in the fluid channel.

Initially, this considerably reduces the risk of bubble formation in the particle fluid so that the danger of clogging the microchannels is also reduced or excluded. Another particular advantage of the invention consists in that the fluidics of the microfluidic system can have a considerably simpler configuration. The reason for this is that it is much easier to hold the particle at or in the through opening. When supplying chemicals for opening the cell in the fluid channel, or compounds to be applied to the cell from the intracellular side, no complicated control for maintaining the necessary pressure differential is required, either. The overpressure in the receptacle can rather be controlled easily in such a manner that the particle remains securely fixed in or at the through opening during the entire measurement or several measurements performed with respect to the particle.

To be able to easily generate pressure in the receptacle by means of a pressure generation device, the receptacle is preferably adapted to be closed. Thereby, it is possible to easily generate pressure in the receptacle by means of a pressure piston. Apart from a supply opening for supplying the particle suspension, which may have a conduit or the like connected thereto, the receptacle comprises, if necessary, a discharge opening for discharging the particle suspension as well as for passing through a cleaning fluid or the like.

The at least one through opening provided in the substrate preferably has a smaller cross section than the particle to be analyzed. The particles in the fluid may possibly have different cross sections so that a portion of the particles is able to flow through the passage opening until a particle closes the through opening and is held thereat or therein.

In a particularly preferred embodiment, the substrate comprises several through openings. Thereby, it is possible to simultaneously position several particles at different through openings by applying pressure in the receptacle.

This further allows the simultaneous analysis of all particles positioned at one through opening ,which may be preferred in e.g. high-throughput measurements.

It may be also preferred that the fluid channel comprises several chambers, each chamber having a through opening allocated thereto. Via supply conduits, a chemical substance for opening the cell, for example, may be supplied to the individual chambers, if necessary, independently of neighboring chambers. If necessary, individual chambers or all of them are adapted to be fluidly connected via valve means. Thereby, it is possible to supply, e.g., a chemical for opening the cells simultaneously to all chambers, to subsequently close the valve means again and to perform different analyses in the individual chambers. Further, e.g., a pressure pulse for opening the cell can be generated in a chamber via the supply means. Opening the cell is also possible because of the pressure differential between receptacle and the corresponding chamber of the fluid channel only.

In another preferred embodiment, a positioning means for supporting the positioning of the particle in or at the through opening due to the fluid flow may additionally be provided. These may be flow elements, e.g., which guide the fluid flow and thus the particles toward a through opening. In particular, the positioning means may comprise at least one annular electrode surrounding the through opening. The annular electrode has an inner diameter that is larger than the outer dimensions of the particle so that the annular electrode does not contact the particle, particularly the biological cell. This avoids damage to the particle. Because of the electric field produced by the annular electrode, the precision of the positioning of the particle in or at the through opening can be improved. In addition to or instead of an annular electrode, additional secondary annular or differently shaped electrodes, e.g, semicircle or rectangular shaped electrodes for positioning the particle, particularly the biological cell, can be provided via electrophoresis or dielectrophoresis..

By means of the microfluidic measuring system according to the invention, it is thus possible to effect a good automatization of the patch clamp measurement. Another advantage of the invention consists in that the pressure control is remarkably easier. The exchange of intracellular liquids as well as the removal of substrates from the fluid channel, if necessary, is remarkably simplified. The microfluidic system according to the invention is particularly suitable for the patch clamp measurement but may also be used for the individual characterization such as the impedance or the use of field effect transistors by means of other electric measuring methods. It has been shown that semiconductor devices such as field effect transistors (FET's) [see e.g. Appl. Phys. A 66, 459-463 (1998)] can be coupled to live biological cells, preferably the surfaces of the FET's being modified with ultra-thin organic layers to ensure biocompatibility. These devices are capable of converting a biological signal into an electrical response because an electrical field created by a living cell such as a neuron can affect the electron population in the semiconductor. Positioning FET's in close proximity to individual cells enables to measure the cells response to drugs, hormones and other compounds. Field effect transistors can both be produced in a suitable polymer or using standard semiconductor technology. In a preferred embodiment the microfluidic system to record electrical signals of single cells or few cells comprises semiconductor materials such as Si or a semiconducting polymer. Those materials as well as glass can also be used to realize electrode configurations, e.g. four terminal configurations, suitable for high resolution impedance measurements of single cells [see e.g. WO002005098395A1]. In addition to the two electrodes 56 and 58 shown in fig. 5, two further electrodes close to the opening 36 on the substrate (hatched area) can be used for such an electrode configuration. A whole frequency spectrum ranging e.g. from the Hertz or even sub-Hertz range to a few 10 kHz or even to the MHz range can be recorded, whereas the lower and higher frequency range yields more sensitive measures for membrane related processes and those of the cell interior, respectively. For many purposes, especially for higher throughput, measurements of the impedance at a few frequencies and evaluating e.g. ratios of the impedances at those frequencies is sufficient for characterisation [see e.g. US020050114041A1 Method for discrimination of particles in a flow cytometer].

In a simple manner, the microfluidic measuring system, which is operated at overpressure according to the invention, may further be connected with available systems for the preceding particle selection or particle cleaning. This is particularly advantageous with apparatus using injection pumps in the "push" mode.

Another essential advantage of the microfluidic system according to the invention is that a high-resistance sealing between the particle and the through opening can be achieved because of the use of overpressure. Thus, the so-called giga seal is able to be easily realized by the invention. This means that the electric current between the receptacle and the fluid channel is extremely low (typical electrical resistance > 10⁹ Ohm).

Another advantage of the microfluidic system according to the invention consists in that it is possible to add fluids, e.g. chemicals for opening the cell, via the fluid channel. This is advantageous since an addition via the receptacle is disadvantageous because it is difficult for the liquid to permeate through the membrane.
Hereinafter, the invention is explained in detail on the basis of a preferred embodiment with respect to the accompanying drawings.

In the Figures:
Figs. 1
   and 2 are schematic diagrams of a microfluidic system according to prior art,
Figs. 3
   and 4 are schematic diagrams of a preferred embodiment of the invention,
Figs.
   5-7 are detail illustrations of the through opening with a cell in different states,
Fig, 8 is a schematic sectional view of a substrate 34 having several chambers, and
Fig. 9 is a schematic sectional view of a substrate 34 having one chamber with several through openings.
Fig. 10 is a schematic view of a positioning means for supporting the positioning of the particle in or at the through opening

In the general representation of the invention according to Figs. 3 and 4, a receptacle 30 contains a liquid having particles suspended therein. A cell 32 is illustrated as a particle in an exemplary manner. The receptacle 30 may include a supply opening 31 for supplying cell suspension and, if necessary, a discharge opening and corresponding valves. It is also possible to supply and discharge cleaning liquids for cleaning the receptacle via the openings. A side wall 34 of the receptacle 30 is configured as a substrate with a through opening 36. Further, a pressure generation means 38 is connected with the receptacle 30. In the illustrated embodiment, the pressure generation means 38 comprises a piston 42 that is displaceable within a cylinder 40. For generating pressure, particularly for increasing the pressure, within the receptacle 30, the piston 42 is moved in the direction of an arrow 44.

At an outer side 46 of the substrate 34, a fluid channel 48 is arranged. Preferably, the fluid channel 48 is configured as a closed portion which, if necessary, comprises particularly two openings opposite each other and being adapted to be closed by valves 50.

By moving the piston in the direction of the arrow 44, a flow indicated by the arrows 52 is generated. Fluid enters the fluid channel through the through opening 36 and flows in the direction of the arrows 54 when the valve 50 is open and the optional supply opening 31 and discharge opening/valves are closed.. This occurs until a cell 32 comes into or to the through opening 36 and closes the latter (Fig. 4). In this situation, a pressure pᵢ is produced within the receptacle because a force acting in the direction of the arrow 44 is still exerted onto the piston 42. This pressure is greater than the external pressure pₑ prevailing in the fluid channel 48.

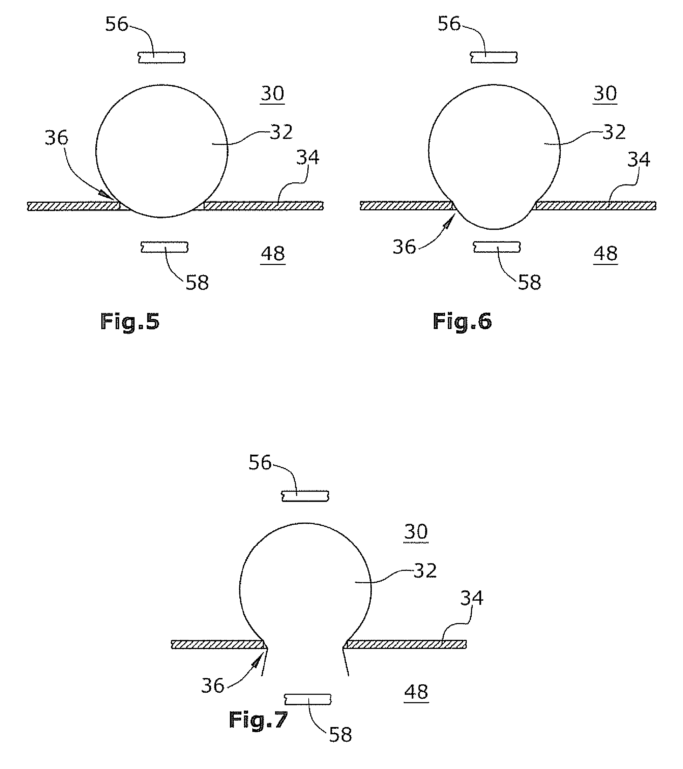

For analyzing the cell, two electrodes 56,58 are provided (Figs. 5-7) which are not illustrated in Fig. 4 for reasons of clarity. The electrode 56 is arranged within the receptacle 30 and the electrode 58 is arranged within the fluid channel 48. The two electrodes are connected with an evaluating means. They may be arranged so as to be opposite each other, the through opening 36 being located between the two electrodes 56,58.

The process of approach and the point of time t0 at which the cell reaches the through opening 36 (see Fig. 5) can be detected electrically by measuring the resistance or impedance between e.g. the electrodes 58 and 59 for at least one frequency in the kHz or MHz range. In further embodyments a pressure sensor or a microscope is used to detect the time t0. By increasing the pressure pᵢ, the cell is deformed, as is shown schematically in Fig. 6. As soon as the pressure differential pᵢ - pₑ exceeds a cell type dependent critical value, the cell membrane breaks, i.e., the interior of the cell 32 opens toward the fluid channel 48. Cell deformation and membrane break can be monitored and /or controlled by resistance or impedance measurements. This is especially advantageous for automated multichannel cell measurements. Further, it is possible to conduct a chemical via the valves 50 which opens the cell. The different means to open the cell 32 may also be combined, if necessary.

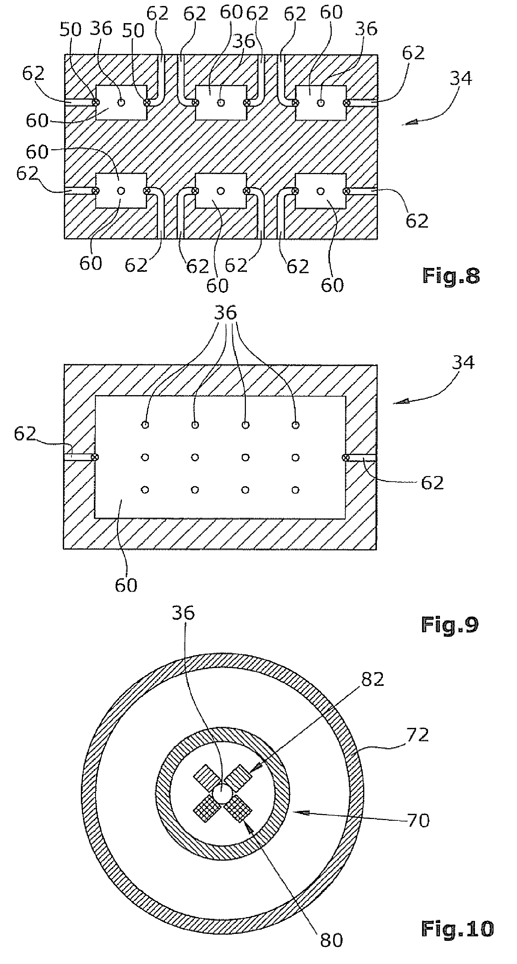

Fig. 8 shows an embodiment of a substrate 34 in sectional view, the fluid channel 48 comprising several chambers 60. The chambers 60 may be individual fluid channels being connected to conduits 62 each. If necessary, neighboring chambers 60 may be directly connected via channels. Each chamber comprises a through opening 36 establishing a connection between the receptacle 30 and the chamber 60.

Fig. 9 shows a further embodiment of a substrate 34 in sectional view. In this embodiment the channel 48 comprises only one chamber 60. The chamber 60 is connected to conduits 62. The chamber 60 comprises a number of through openings 36 establishing a connection between the receptacle 30 and the chamber 60.

Furthermore, a combination of the embodiments shown in Fig. 8 and 9 are possible so that the fluid channel 48 comprises several chambers 60, whereby the chambers may have a varying number of through openings 36.

Fig. 10 shows an embodiment of a positioning means for supporting the positioning of the particle, comprising two annular electrodes 70, 72 surrounding the through opening 36. The inner annular electrode 70 has an inner diameter that is larger than the outer dimensions of the particle so that the annular electrode does not contact the particle, particularly the biological cell. In addition to the annular electrodes, additional differently shaped electrodes 80, 82, for cell manipulation and/or characterisation,e.g. by impedance measurements, are shown. These electrodes are arranged near or at the through opening. Here four electrodes are shown, suited for impedance measurements. Two of theses electrodes 80 are providing current and the other two 82 are suited for voltage measurements.

Furthermore the invention is related to a process of easily capture and analyzing particles, particularly biological cells, membranes (particularly lipid membranes), membrane fragments, vesicles (particularly lipid vesicles), lipids, cell fragments, beads (particularly labeled beads).

The process for the analysis of particles according to the invention comprises the following steps:
1) Filling of receptacle 30 and fluid channel 48 with electrolyte solution (or buffer). As pre-step, a priming of the substrate may be required.
2) Injection of cells e. g. via opening 31 into receptacle 30
3) Open valves 50
4) Application of overpressure by moving the piston 42 in the direction of the arrow 44 (see Fig. 3 and 4). Ideally one cell is positioned in or at the through opening 36. This can be detected via a pressure increase in receptacle 30, via an increase in electrical resistance between the electrodes, via a change in capacitance, or other electrical or pressure measures. Supporting electrodes may be used to position the cell.
5) Holding or increase of the overpressure, to generate a high-resistance seal (e.g. a mega-seal or giga-seal). The pressure needs to be carefully controlled in this step. In a preferred embodiment, pressure ramps may be beneficial to generate good quality seals. The cell is now in the cell-attached configuration.
6) Abrupt significant increase of the pressure to generate a whole-cell configuration. Alternatively, a voltage pulse between the electrodes can be used to generate the whole cell configuration, or flushing a pore-forming agent through fluid channel 48. The established whole-cell configuration is detected via an increase of the capacitance between the electrodes. A preferred embodiment uses voltage pulses of e.g. 10 mV, with a duration of e.g. 5 ms, or at a frequency of approx 10 kHz, to probe the capacitance, Application of an agent can alternatively be controlled by a delay-time.
7) Setting pressure in receptacle 30 to a defined value to hold the cell (pᵢ is slightly higher than pₑ) .
8) Flush receptacle 30 to flush away excess cells that are not in the measurement configuration.
9) Actual start of measurements.
10) Flushing of test agents, preferred into fluid channel 48, or alternatively into receptacle 30. Measurement of cell response to agents.
11) Overpressure via fluid channel 48, or underpressure in receptacle 30, is used to rinse cells away.

## Claims

1. A microfluidic system for analyzing particles, particularly biological cells, comprising
a receptacle (30) adapted to be supplied with a fluid having particles (32) suspended therein,
a substrate with at least one through opening and forming at least a part of a side wall (34) of the receptacle (30),
a fluid channel (48) arranged at an outer side (46) of the substrate (34),
a pressure generation means (38) for generating a flow through the through opening (36) and being connected to the receptacle (30), and
electrodes (56,58) being arranged on different sides of the substrate (34), for performing electric measurements with respect to a particle (32) arranged in or at the through opening (36)..

2. The microfluidic system according to claim 1, **characterized in that** the pressure generation means (38) generates such a fluid flow that a particle (32) is arranged in or at the through opening (36) and held during the measurement.

3. The microfluidic system according to claim 1 or 2, **characterized in that** the pressure generation means (38) generates an overpressure in the receptacle (30) with respect to the fluid channel (48) during or before the measurement to fix the particle in or at the through opening (36).

4. The microfluidic system according to one of claims 1-3 , **characterized in that** the pressure generation means (38) generates an overpressure in the receptacle (30) with respect to the fluid channel (48) to generate a giga-seal or a mega-seal.

5. The microfluidic system according to one of claims 1-4, **characterized in that** the cell is opened into the whole-cell configuration.

6. The microfluidic system according to one of claims 1 - 5, **characterized in that** the receptacle (30) is adapted to be closed for generating pressure.

7. The microfluidic system according to one of claims 1 - 6, **characterized in that** the through opening (36) has a smaller cross section than the particle (32) to be analyzed.

8. The microfluidic system according to one of claims 1 - 7, **characterized in that** the fluid channel (48) comprises several through openings (36).

9. The microfluidic system according to one of claims 1 - 7, **characterized in that** the fluid channel (48) comprises several chambers (60), each chamber (60) having at least one through opening (36) allocated thereto.

10. The microfluidic system according to claim 9, **characterized in that** the chambers (60) are adapted to be fluidly separated from each other or are fluidly separated from each other.

11. The microfluidic system according to claim 9 or 10, **characterized in that** the chambers (60), particularly valve means (50), are fluidly connectable.

12. The microfluidic system according to one of claims 1 - 11, **characterized by** a supply means for supplying chemical and/or biological fluids into the fluid channel (48) or individual chambers (60) of the fluid channel (48).

13. The microfluidic system according to one of claims 1 - 12, **characterized in that** the electrodes (56,58) have such a distance to the through opening (36) that a particle (32) positioned in the through opening (36) does not contact any of the two electrodes (56,58).

14. The microfluidic system according to one of claims 1 - 13, **characterized by** a positioning means for supporting the positioning of the particle (32) in or at the through opening (36) because of the fluid flow.

15. The microfluidic system according to claim 14, **characterized in that** the positioning means comprises an annular electrode surrounding the through opening (36).

16. The microfluidic system according to one of claims 1 - 15, **characterized by** a semiconductor device, such as a field effect transistor (FET), at the through opening (36).

17. The microfluidic system according to one of claims 1 - 16, **characterized by** at least two further electrodes being arranged on different sides of the particle.

18. A process for the analysis of particles comprising the following steps:
- arranging a particle (32) in or at the through opening (36) of a substrate by of application of overpressure
- performing electric measurements with respect to particle (32).

19. Process according to claim 18, **characterised in that** the overpressure is hold or increased to generate a high-resistance seal (e.g. a mega-seal or giga-seal).

20. Process according to one of claims 18-19, **characterised in that** a whole-cell configuration is generated by abrupt significant increase of the pressure or by a voltage pulse between the electrodes (56, 58) or by flushing a pore-forming agent through fluid channel (48).

21. Process according to one of claims 18-20, **characterised in that** the electric measurement is a patch-clamp measurement and/or an impedance measurement and/or the measurement of electrical fields generated by particle (32) by field effect transistors.

22. Process according to one of claims 18-21, whereby the process is performed by use of the microfluidic system according to one of claims 1-17.
